# EUROPEAN PATENT APPLICATION

(11) **EP 4 628 589 A1**
(43) Date of publication of application: **08.10.2025**
(21) Application number: 23912957.0
(22) Date of filing: 28.12.2023
(51) Int. Cl.: C12N 15/77, C12N 9/00, C12P 13/22

(54) **MICROORGANISM INTO WHICH HETEROLOGOUS GLUTAMINE SYNTHETASE IS INTRODUCED, AND METHOD FOR PRODUCING L-TRYPTOPHAN BY USING SAME**

(30) Priority: 28.12.2022 KR 20220187715
(71) Applicant: CJ CheilJedang Corporation, Seoul 04560 (KR)
(72) Inventor: PARK, Soe-hee, Seoul 04560 (KR); JUNG, Moo Young, Seoul 04560 (KR); PARK, Seul-Gi, Seoul 04560 (KR); SHIM, Jihyun, Seoul 04560 (KR); LEE, Sumin, Seoul 04560 (KR)
(74) Representative: Meissner Bolte Partnerschaft mbB
(86) International application number: PCT/KR2023/021782
(87) International publication number: WO 2024/144283

(57) **Abstract**

The present disclosure relates to a microorganism, into which a foreign glutamine synthetase is introduced, and a method of producing L-tryptophan using the same.

## Description

### [Technical Field]

The present disclosure relates to a microorganism, into which a foreign glutamine synthetase is introduced, and a methEP 23 901 020.0
Our Ref.: M/CJG-089-PC/EP
od of producing L-tryptophan using the same.

### [Background Art]

Various studies are being conducted to develop highly efficient microorganisms and fermentation process technologies for the production of L-amino acids and other useful substances. For example, when L-tryptophan is produced, a target substance-specific approach is mainly used, in which the expression of a gene encoding an enzyme involved in L-tryptophan biosynthesis is increased, or in which genes unnecessary for the biosynthesis are removed (US 8945907 B2).

However, as the demand for L-tryptophan increases, it is still necessary to conduct studies to effectively increase the L-tryptophan producing ability.

### [Disclosure]

### [Technical Problem]

The present inventors have confirmed that when a foreign glnA protein is introduced into a microorganism, L-tryptophan producing ability is increased, as compared to an unmodified microorganism, thereby completing the present disclosure.

### [Technical Solution]

An object of the present disclosure is to provide a microorganism of the genus *Corynebacterium,* into which a foreign glnA protein or a polynucleotide encoding the same is introduced.

Another object of the present disclosure is to provide a method of producing L-tryptophan, the method including the step of culturing, in a medium, a microorganism of the genus *Corynebacterium,* into which a foreign glnA protein or a polynucleotide encoding the same is introduced.

Still another object of the present disclosure is to provide a composition for producing L-tryptophan, the composition including a microorganism of the genus *Corynebacterium,* into which a foreign glnA protein or a polynucleotide encoding the same is introduced; a medium in which the microorganism is cultured; or a combination thereof.

### [Advantageous Effects]

A microorganism of the present disclosure is introduced with a foreign glnA protein, thereby having the increased L-tryptophan producing ability, as compared to the existing unmodified microorganism.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a microorganism of the genus *Corynebacterium,* into which a foreign glnA protein or a polynucleotide encoding the same is introduced.

As used herein, the term "glutamine synthetase (glnA)" refers to a protein having the activity of biosynthesizing glutamine from glutamic acid and ammonia. The "glutamine synthetase" may be used interchangeably with "glnA protein", "glnA", etc. Further, it may be a protein which is also called "glutamate-ammonia ligase as a protein having the activity of biosynthesizing glutamine from glutamic acid and ammonia.

An amino acid sequence of the glnA protein may be available in the NCBI GenBank, etc., which is a known database.

For example, the glnA protein of the present disclosure may be derived from a microorganism. The protein may be specifically derived from a microorganism selected from a microorganism of the genus *Aureibacillus,* a microorganism of the genus *Caryophanon,* and a microorganism of the genus *Peribacillus,* and more specifically, may be derived from a microorganism selected from *Aureibacillus halotolerans, Caryophanon tenue,* and *Peribacillus simplex,* but is not limited thereto.

For another example, the amino acid sequence of the glnA protein of the present disclosure may be WP_133580410.1 (GenBank accession number) derived from *Aureibacillus halotolerans,* WP_066543527.1 (GenBank accession number) derived from *Caryophanon tenue,* and WP_063236399.1 (GenBank accession number) derived from *Peribacillus simplex.*

In the present disclosure, the glnA protein may have, include, or consist of any one amino acid sequence of SEQ ID NOS: 1, 3, and 5, or may essentially consist of the amino acid sequence.

In the present disclosure, the glnA protein may include any one amino acid sequence of SEQ ID NOS: 1, 3, and 5, or an amino acid sequence having at least 70% or more, 75% or more, 76% or more, 85% or more, 86% or more, 87% or more, 88% or more, 89% or more, 90% or more, 91% or more, 92% or more, 93% or more, 94% or more, 95% or more, 96% or more, 97% or more, 98% or more, 99% or more, 99.5% or more, 99.7% or more, or 99.9% or more homology or identity thereto. For example, the glnA protein of the present disclosure may include any one amino acid sequence of SEQ ID NOS: 1, 3, and 5, or any one or more selected from the group consisting of amino acid sequences having 90% or more homology or identity thereto. Further, it is apparent that any protein having an amino acid sequence having deletion, modification, substitution, conservative substitution, or addition in part of the sequence may also be included within the scope of the present disclosure as long as it has such homology or identity and exhibits the efficacy corresponding to that of the protein including any one amino acid sequence of SEQ ID NOS: 1, 3, and 5.

Examples thereof include those having sequence addition or deletion that does not alter the function of the protein of the present disclosure at the N-terminus, C-terminus of the amino acid sequence, and/or inside the amino acid sequence, naturally occurring mutation, silent mutation, or conservative substitution.

The "conservative substitution" means substitution of one amino acid with another amino acid having similar structural and/or chemical properties. Such an amino acid substitution may generally occur based on similarity in the polarity, charge, solubility, hydrophobicity, hydrophilicity, and/or amphipathic nature of the residues. Usually, conservative substitution may hardly affect or not affect the activity of proteins or polypeptides.

As used herein, the term 'homology' or 'identity' refers to the degree of similarity between two given amino acid sequences or nucleotide sequences and may be expressed as a percentage. The terms 'homology and identity' may be often used interchangeably.

The sequence homology or identity of conserved polynucleotides or polypeptides may be determined by a standard alignment algorithm, and default gap penalties established by a program to be used may be used together. Substantially, homologous or identical sequences may generally hybridize with each other as a whole or in part under moderate or highly stringent conditions. It is obvious that the hybridization also includes hybridization with a polynucleotide containing usual codons or codons considering codon degeneracy in the polynucleotide.

Whether any two polynucleotide or polypeptide sequences have homology, similarity, or identity may be determined by, for example, a known computer algorithm such as the "FASTA" program using default parameters as in Pearson et al (1988)[Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ET AL.](1988) SIAM J Applied Math 48: 1073). For example, homology, similarity, or identity may be determined using BLAST or ClustalW of the National Center for Biotechnology Information.

The homology, similarity, or identity between polynucleotides or polypeptides may be determined by comparing sequence information using the GAP computer program, for example, Needleman et al. (1970), J Mol Biol. 48:443, as disclosed by Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may include: (1) a unitary matrix (containing a value 1 for identity and a value 0 for non-identity) and the weighted comparison matrix of Gribskov et al(1986) Nucl. Acids Res. 14: 6745, as disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

The glnA protein of the present disclosure may be encoded by *glnA* gene.

For example, the *glnA* gene may be a polynucleotide encoding WP_133580410.1 derived from *Aureibacillus halotolerans,* WP_066543527.1 derived from *Caryophanon tenue,* or WP_063236399.1 derived from *Peribacillus simplex,* but is not limited thereto. For another example, the *glnA* gene may be NZ_SNYJ01000007.1 derived from *Aureibacillus halotolerans, NZ_MASJ01000003.1* derived from *Caryophanon tenue,* or NZ_CP017704.1 derived from *Peribacillus simplex,* but is not limited thereto, and it is apparent that the *glnA* gene includes glnA genes derived from various sources, which encode the protein having the glutamine synthetase activity.

As used herein, the term "polynucleotide" is a DNA or RNA strand having a certain length or more as a polymer of nucleotides in which nucleotide monomers are connected in a long chain by covalent bonds, and more specifically, refers to a polynucleotide fragment encoding the protein.

The polynucleotide encoding the glnA protein of the present disclosure may include a nucleotide sequence encoding any one amino acid sequence of SEQ ID NOS: 1, 3, and 5. In one embodiment of the present disclosure, the polynucleotide of the present disclosure may have or may include any one nucleotide sequence of SEQ ID NOS: 2, 4, and 6. Further, the polynucleotide of the present disclosure may consist of or may essentially consist of any one nucleotide sequence of SEQ ID NOS: 2, 4, and 6. Specifically, the *glnA* gene may be encoded by a polynucleotide represented by any one nucleotide sequence of SEQ ID NOS: 2, 4, and 6.

In the polynucleotide of the present disclosure, various modifications may be made in the coding region as long as the amino acid sequence of the glutamine synthetase of the present disclosure is not changed in consideration of codon degeneracy or codons preferred in organisms that are intended to express the glutamine synthetase of the present disclosure. Specifically, the polynucleotide of the present disclosure may have or may include a nucleotide sequence having 70% or more, 75% or more, 76% or more, 85% or more, 90% or more, 95% or more, 96% or more, 97% or more, 98% or more, or 99% or more homology or identity to any one sequence of SEQ ID NOS: 2, 4, and 6, or may consist of or may essentially consist of a nucleotide sequence having 70% or more, 75% or more, 76% or more, 85% or more, 90% or more, 95% or more, 96% or more, 97% or more, 98% or more, or 99% or more homology or identity to any one sequence of SEQ ID NOS: 2, 4, and 6, but is not limited thereto.

Further, the polynucleotide of the present disclosure may include a probe that may be prepared from a known gene sequence, for example, may include a sequence without limitation as long as it is a sequence that is able to hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof include a condition in which polynucleotides having higher homology or identity, i.e., polynucleotides having 70% or more, 75% or more, 76% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically, twice to three times at a salt concentration and temperature equivalent to 60°C, 1X SSC, 0.1% SDS, specifically, 60°C, 0.1X SSC, 0.1% SDS, more specifically 68°C, 0.1X SSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also include substantially similar nucleic acid sequences as well as isolated nucleic acid fragments that are complementary to the entire sequence.

Specifically, a polynucleotide having homology or identity to the polynucleotide of the present disclosure may be detected using a hybridization condition including a hybridization step at a Tm value of 55°C and the above-described conditions. Further, the Tm value may be 60°C, 63°C, or 65°C, but is not limited thereto, and may be appropriately adjusted by those skilled in the art according to the purpose.

**The** appropriate stringency to hybridize the polynucleotide depends on the length and degree of complementarity of the polynucleotide, and the variables are well known in the art (e.g., J. Sambrook et al., supra).

As used herein, the term "microorganism (or strain)" includes all of wild-type microorganisms or naturally or artificially genetically modified microorganisms, and it may be a microorganism in which a specific mechanism is strengthened or weakened due to insertion of a foreign gene or an activity enhancement or attenuation of an endogenous gene, and it may be a microorganism including genetic modification for production of a polypeptide, protein, or product of interest.

Therefore, examples of the microorganism of the present disclosure may include a recombinant microorganism which is introduced with a foreign glnA protein having increased glutamine synthetase activity, as compared to that of the wild-type microorganism of the genus *Corynebacterium,* and/or a recombinant microorganism having a genetic modification which enhances the activity of glnA protein, as compared to the endogenous activity thereof.

The microorganism of the present disclosure may have the increased activity of glnA protein, as compared to the endogenous activity thereof. For example, the microorganism may be a microorganism having improved L-tryptophan producing ability by introducing the activity of the foreign glnA protein. The microorganism may be a microorganism having the enhanced activity of glnA protein by introducing the activity of the foreign glnA protein which is not endogenously expressed.

The enhanced activity of glnA protein is caused by introduction of the glnA protein which is not endogenously expressed, and may refer to exhibiting the activity of the foreign glnA protein which is not originally possessed. Alternatively, the enhanced activity of glnA protein may exhibit the increased activity, as compared to that of the endogenous glnA protein.

For example, the microorganism of the present disclosure may have the enhanced activity of glutamine synthetase in the microorganism by introducing the foreign glnA protein having the increased glutamine synthetase activity, as compared to that of the wild-type microorganism of the genus *Corynebacterium,* but is not limited thereto.

As used herein, the term "enhancement" of a polypeptide (e.g., including proteins specified by the name of each enzyme) means that the activity of a polypeptide is increased, as compared to the endogenous activity. The enhancement may be used interchangeably with terms such as activation, up-regulation, overexpression, and increase, etc. Here, activation, enhancement, up-regulation, overexpression, and increase may include both exhibiting activity that was not originally possessed and exhibiting improved activity as compared to the endogenous activity or activity before modification. The "endogenous activity" means the activity of a specific polypeptide originally possessed by a parent strain before the trait is changed or an unmodified microorganism, when the trait is changed by genetic variation due to natural or artificial factors. This may be used interchangeably with "activity before modification". The fact that the activity of a polypeptide is "enhanced", "up-regulated", "overexpressed", or "increased" as compared to the endogenous activity means that the activity of the polypeptide is improved as compared to the activity and/or concentration (expression level) of a specific polypeptide originally possessed by a parent strain before the trait is changed or an unmodified microorganism.

The enhancement may be achieved through the introduction of a foreign polypeptide or the enhancement of endogenous activity and/or concentration (expression level) of the polypeptide. The enhancement of the activity of a polypeptide may be confirmed by an increase in the degree of activity and the expression level of the corresponding polypeptide or in the amount of the product produced from the corresponding polypeptide.

With respect to the objects of the present disclosure, the microorganism of the present disclosure may have the enhanced L-tryptophan producing ability due to the enhanced activity of glutamine synthetase in the microorganism by introducing the foreign glnA protein having the increased activity of glutamine synthetase, as compared to that of the wild-type microorganism of the genus *Corynebacterium.* The unmodified microorganism not introduced with the foreign glnA protein, which is a target strain for comparing whether or not the L-tryptophan producing ability or the glutamine synthetase activity is increased, may be an L-tryptophan-producing CM05-9157 strain (US 2023-0134555 A1) and a CM05-9157 strain introduced with the glnA protein derived from the wild-type *Corynebacterium glutamicum,* but is not limited thereto.

For the enhancement of the activity of the polypeptide, various methods well known in the art may be applied, and the method is not limited as long as the activity of the polypeptide of interest may be enhanced as compared to that of the microorganism before being modified. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the activity enhancement of the polypeptide of the present disclosure may be:
1) increase in the intracellular copy number of the polynucleotide encoding the polypeptide;
2) replacement of a gene expression regulatory region on a chromosome encoding the polypeptide with a sequence exhibiting strong activity;
3) modification of a nucleotide sequence encoding an initiation codon or 5'-UTR region of the gene transcript encoding the polypeptide;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select and to modify or chemically modify the exposed site; or
9) a combination of two or more selected from 1) to 8), but is not particularly limited thereto.

More specifically:
1) The increase in the intracellular copy number of the polynucleotide encoding the polypeptide may be achieved by the introduction of, into a host cell, a vector which may replicate and function independently of the host and to which the polynucleotide encoding the corresponding polypeptide is operably linked. Alternatively, the increase may be achieved by the introduction of one copy or two or more copies of the polynucleotide encoding the corresponding polypeptide into a chromosome of a host cell. The introduction into the chromosome may be performed by introducing, into a host cell, a vector capable of inserting the polynucleotide into a chromosome of the host cell, but is not limited thereto. The vector is as described above.
2) The replacement of a gene expression regulatory region (or expression control sequence) on a chromosome encoding the polypeptide with a sequence exhibiting strong activity may be, for example, occurrence of variation in a sequence due to deletion, insertion, nonconservative or conservative substitution, or a combination thereof, or replacement with a sequence exhibiting stronger activity so that the activity of the expression regulatory region is further enhanced. The expression regulatory region may include, but is not particularly limited to, a promoter, an operator sequence, a sequence encoding a ribosome binding site, a sequence controlling the termination of transcription and translation, etc. For example, the replacement may be to replace the original promoter with a strong promoter, but is not limited thereto.
   Examples of known strong promoters include a PlysCm1 promoter (US 2023-0134555 A1), CJ1 to CJ7 promoters (US 7662943 B2), a lac promoter, a trp promoter, a trc promoter, a tac promoter, a lambda phage PR promoter, a PL promoter, a tet promoter, a gapA promoter, a SPL7 promoter, a SPL13(sm3) promoter (US 10584338 B2), an O2 promoter (US 10273491 B2), a tkt promoter, an yccA promoter, etc., but is not limited thereto.
3) The modification of a nucleotide sequence encoding an initiation codon or 5'-UTR region of the gene transcript encoding the polypeptide may be, for example, substitution with a nucleotide sequence encoding another start codon having a higher polypeptide expression rate as compared to an endogenous start codon, but is not limited thereto.
4) and 5) The modification of the amino acid sequence or the polynucleotide sequence may be occurrence of variation in the sequence due to deletion, insertion, nonconservative or conservative substitution of the amino acid sequence of the polypeptide or the polynucleotide sequence encoding the polypeptide, or a combination thereof, or replacement with an amino acid sequence or polynucleotide sequence improved to exhibit stronger activity or an amino acid sequence or polynucleotide sequence improved to be more active so that the activity of the polypeptide is enhanced, but is not limited thereto. The replacement may be specifically performed by inserting a polynucleotide into a chromosome by homologous recombination, but is not limited thereto. The vector used here may further include a selection marker for the confirmation of chromosome insertion.
6) The introduction of a foreign polynucleotide exhibiting the activity of the polypeptide may be introduction of a foreign polynucleotide encoding a polypeptide exhibiting activity identical or similar to that of the polypeptide into a host cell. There is no limitation on its origin or sequence as long as the foreign polynucleotide exhibits activity identical or similar to that of the polypeptide. The method used in the introduction may be performed by appropriately selecting a known transformation method by those skilled in the art. As the introduced polynucleotide is expressed in a host cell, the polypeptide may be produced, and the activity thereof may be increased.
7) The codon optimization of the polynucleotide encoding the polypeptide may be codon optimization of an endogenous polynucleotide so as to increase transcription or translation in a host cell, or codon optimization of a foreign polynucleotide so as to perform optimized transcription and translation in a host cell.
8) The analysis of the tertiary structure of the polypeptide to select and to modify or chemically modify the exposed site may be, for example, to determine a template protein candidate according to the degree of similarity of the sequence by comparing the sequence information of a polypeptide to be analyzed with a database storing the sequence information of known proteins, to confirm the structure based on this, and to modify or chemically modify the exposed site to be modified or chemically modified.

Such enhancement of the polypeptide activity may be an increase in the activity or concentration expression level of the corresponding polypeptide, based on the activity or concentration of the polypeptide expressed in a wild-type or a microbial strain before being modified, or an increase in the amount of a product produced from the corresponding polypeptide, but is not limited thereto.

For example, the enhancement of the glutamine synthetase activity of the present disclosure may be enhancement by introduction of a foreign polynucleotide exhibiting the glutamine synthetase activity. For another example, the enhancement of the glutamine synthetase activity of the present disclosure may be enhancement due to replacement of a promoter of the gene encoding glutamine synthetase or enhancement due to modification of the promoter. For still another example, the enhancement of the glutamine synthetase activity of the present disclosure may be enhancement due to modification of the nucleotide sequence encoding the start codon of the gene transcript encoding glutamine synthetase. For still another example, the enhancement may be a combination of the above modifications, but is not limited thereto.

In the microorganism of the present disclosure, partial or entire modification of the polynucleotide may be induced by (a) homologous recombination using a vector for chromosome insertion in the microorganism or genome editing using engineered nuclease (e.g., CRISPR-Cas9) and/or (b) treatment with light such as ultraviolet rays and radiation, and/or chemicals, but is not limited thereto. A method of modifying a part or the entirety of the gene may include a method of using a DNA recombination technology. For example, by introducing a nucleotide sequence or vector including a nucleotide sequence homologous to the gene of interest into the microorganism to cause homologous recombination, a part or the entirety of the gene may be deleted. The nucleotide sequence or vector to be introduced may include a dominant selection marker, but is not limited thereto.

The vector of the present disclosure may include a DNA construct including a polynucleotide sequence encoding a polypeptide of interest which is operably linked to a suitable expression regulatory region (or expression control sequence) so that the polypeptide of interest may be expressed in a suitable host. The expression regulatory region may include a promoter capable of initiating transcription, any operator sequence for controlling the transcription, a sequence encoding a suitable mRNA ribosome binding site, and a sequence controlling termination of transcription and translation. The vector may be transformed into a suitable host cell, and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pBR system, pUC system, pBluescript II system, pGEM system, pTZ system, pCL system, pET system, etc. may be used as a plasmid vector. Specifically, pDZ, pDC, pDCM2, pACYC177, pACYC184, pCL, pECCG117, pUC19, pBR322, pMW118, pCC1BAC vector, etc. may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further include a selection marker for the confirmation of chromosome insertion. The selection marker is for selecting the cells transformed with vectors, i.e., for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector including a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the host cell or located outside the chromosome as long as it may be expressed in the host cell. Further, the polynucleotide includes DNA and/or RNA encoding a polypeptide of interest. The polynucleotide may be introduced in any form as long as it may be introduced into a host cell and expressed. For example, the polynucleotide may be introduced into a host cell in the form of an expression cassette, which is a gene construct containing all elements required for self-expression. The expression cassette may usually include a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the polynucleotide sequence is functionally linked to a promoter sequence that initiates and mediates transcription of the polynucleotide encoding the target polypeptide of the present disclosure.

In another embodiment of the present disclosure, the microorganism of the present disclosure may be a microorganism having the L-tryptophan producing ability.

The microorganism of the present disclosure may be a microorganism having the improved L-tryptophan producing ability.

The microorganism of the present disclosure may have the enhanced glutamine synthetase activity. Specifically, the microorganism of the present disclosure may be a microorganism in which the foreign glnA protein or the *glnA* gene encoding the same is enhanced; or a microorganism genetically modified to further enhance the foreign glnA protein or the *glnA* gene encoding the same, but is not limited thereto. The microorganism in which the foreign glnA protein or the *glnA* gene encoding the same is enhanced may have the increased glutamine synthetase activity, as compared to the wild-type or microorganism of the genus *Corynebacterium* before being modified. For example, the microorganism of the present disclosure may be a recombinant microorganism.

The microorganism of the present disclosure may be a microorganism naturally having the L-tryptophan producing ability, or a microorganism in which the L-tryptophan producing ability is increased or imparted by introducing the foreign glnA protein or the polynucleotide encoding the same into a parent strain without the L-tryptophan producing ability, but is not limited thereto.

With respect to the objects of the present disclosure, the recombinant microorganism of the present disclosure may be a microorganism having the increased L-tryptophan producing ability by introducing the foreign glnA protein or the polynucleotide encoding the same into a natural wild-type microorganism or into a microorganism producing L-tryptophan by including the protein having endogenous glutamine synthetase activity or the polynucleotide encoding the same, as compared to the natural wild-type microorganism or the microorganism producing L-tryptophan by including the protein having endogenous glutamine synthetase activity or the polynucleotide encoding the same, but is not limited thereto. For example, the natural wild-type microorganism or the microorganism producing L-tryptophan by including the protein having endogenous glutamine synthetase activity or the polynucleotide encoding the same may be a target strain for comparing whether or not the L-tryptophan producing ability or the glutamine synthetase activity is increased, as described above, but is not limited thereto.

For example, the recombinant strain having the increased producing ability may have an L-tryptophan producing ability of about 1% or more, specifically, about 2% or more, about 5% or more, about 7.5% or more, about 10% or more, about 12.5% or more, about 15% or more, about 17.5% or more, about 20% or more, about 21% or more, about 22% or more, about 23% or more, about 24% or more, or about 25% or more (the upper limit is not particularly limited, but may be, for example, about 200% or less, about 150% or less, about 100% or less, about 50% or less, about 40% or less, about 30% or less, about 20% or less, or about 15% or less), as compared to that of the parent strain before modification or unmodified microorganism, but the increased amount is not limited thereto as long as the producing ability has an increased amount of a + value, as compared to the producing ability of the parent strain before modification or the unmodified microorganism. In another example, the microorganism having the increased producing ability may have an L-tryptophan producing ability of about 1.01 times or more, about 1.02 times or more, about 1.05 times or more, about 1.075 times or more, about 1.1 times or more, about 1.125 times or more, about 1.15 times or more, about 1.175 times or more, about 1.2 times or more, about 1.21 times or more, about 1.22 times or more, about 1.23 times or more, about 1.24 times or more, or about 1.25 times or more (the upper limit is not particularly limited, but may be, for example, about 10 times or less, about 5 times or less, about 3 times or less, about 2 times or less), as compared to that of the parent strain before modification or the unmodified microorganism, but is not limited thereto.

Such producing ability may be evaluated by measuring the production amount of the target product which is obtained by culturing in a medium. The evaluation may be performed by measuring the production amount of the target product using an appropriate method known in the art. For example, high performance liquid chromatography (HPLC), gas chromatography (GC), gas chromatography-mass spectrometry (GC/MS), liquid chromatography-mass spectrometry (LC/MS), gel permeation chromatography (GPC), or a combination thereof may be used, and the production amount of the target product may be measured using an appropriate method known in the art.

As used herein, the term "unmodified microorganism" may refer to a wild-type strain or a native strain as it is, or a strain before transformation due to genetic mutation caused by natural or artificial factors, not excluding strains including mutations that may naturally occur in microorganisms. For example, the unmodified microorganism may refer to a strain in which the glutamine synthetase or the polynucleotide encoding the same described herein is not enhanced, or a strain before the enhancement thereof. The "unmodified microorganism" may be used interchangeably with "strain before modification", "microorganism before modification", "unvaried strain", "unmodified strain", "unvaried microorganism", or "reference microorganism".

For still another example of the present disclosure, the microorganism of the present disclosure may be *Corynebacterium glutamicum, Corynebacterium stationis, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* specifically, *Corynebacterium glutamicum,* but is not limited thereto.

For still another example of the present disclosure, the recombinant microorganism of the present disclosure may be a microorganism in which the L-tryptophan producing ability is enhanced by additionally enhancing the activity of some of the proteins involved in the L-tryptophan biosynthesis pathway, or by additionally diminishing the activity of some of the proteins involved in the L-tryptophan degradation pathway.

In one embodiment, the recombinant microorganism of the present disclosure may be a microorganism in which, for enhancing the L-tryptophan biosynthetic pathway, the activities of the gene in the competitive pathway, the regulator in the directional pathway of L-tryptophan operon, the gene for introducing L-tryptophan, and the gene for introducing and decomposing L-tryptophan are diminished or inactivated, and/or the activity of the L-tryptophan operon is overexpressed.

In another embodiment, the recombinant microorganism of the present disclosure may be a microorganism in which a regulatory gene (trpR) of a tryptophan synthesis enzyme group that represses the expression of L-tryptophan biosynthesis genes (e.g., trpEDCBA) or the activity of Mtr membrane protein that imports extracellular L-tryptophan into a cell may be diminished or removed, as compared to the endogenous activity thereof, but is not limited thereto.

As used herein, the term "diminishment" of the activity of a polypeptide has a concept encompassing all of the reduction of activity or the absence of activity, as compared to the endogenous activity. The diminishment may be used interchangeably with inactivation, deficiency, down-regulation, decrease, reduction, attenuation, or the like.

The diminishment may also include a case where the activity of the polypeptide itself is reduced or eliminated due to the mutation, etc. of the polynucleotide encoding the polypeptide, as compared to the activity of the polypeptide originally possessed by the microorganism; a case where the activity and/or concentration (expression level) of the overall polypeptides in the cell is lower due to the inhibition of the expression of a gene of a polynucleotide encoding the polypeptide or the inhibition of translation into the polypeptide, as compared to the native strain; a case where the expression of the polynucleotide is not made; and/or a case where the polypeptide has no activity even though the polynucleotide is expressed. The "endogenous activity" refers to the activity of a specific polypeptide originally possessed by a parent strain before modification or a wild-type or unmodified microorganism, when transformed by genetic mutation caused by natural or artificial factors. This term may be used interchangeably with the "activity before modification". The "diminishment, inactivation, deficiency, decrease, down-regulation, reduction, or attenuation" of the activity of a polypeptide compared to the endogenous activity thereof means that the activity of the polypeptide is lowered compared to the activity of a specific polypeptide originally possessed by a parent strain before transformation or an unmodified microorganism.

The diminishment of the activity of the polypeptide may be performed by any method known in the art, but is not limited thereto, and may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the diminishment of the activity of the polypeptide of the present disclosure may be achieved by:
1) deletion of the entirety or a part of the gene encoding the polypeptide;
2) modification of an expression control region (or expression control sequence) to reduce the expression of the gene encoding the polypeptide;
3) modification of the amino acid sequence constituting the polypeptide to eliminate or diminish the activity of the polypeptide (e.g., deletion/substitution/addition of one or more amino acids in the amino acid sequence);
4) modification of the gene sequence encoding the polypeptide to eliminate or diminish the activity of the polypeptide (e.g., deletion/substitution/addition of one or more nucleotides in the nucleotide sequence of the polypeptide gene so as to encode the modified polypeptide of which activity is eliminated or diminished);
5) modification of a nucleotide sequence encoding an initiation codon, a Shine-Dalgarno sequence, or 5'-UTR region of the gene transcript encoding the polypeptide;
6) introduction of an antisense oligonucleotide (e.g., antisense RNA) complementarily binding to the gene transcript encoding the polypeptide;
7) addition of a sequence complementary to the Shine-Dalgarno sequence of the gene encoding the polypeptide to the upstream of the Shine-Dalgarno sequence in order to form a secondary structure that makes the attachment of ribosomes impossible;
8) addition of a promoter, which is to be reverse-transcribed, to the 3' end of the open reading frame (ORF) of the gene sequence encoding the polypeptide (reverse transcription engineering, RTE); or
9) combination of two or more selected from 1) to 8), but is not particularly limited thereto.

For example,
1) the deletion of a part or the entirety of the gene encoding the polypeptide may be the elimination of the entirety of the polynucleotide encoding the endogenous target polypeptide in the chromosome, the replacement with a polynucleotide having deletion of some nucleotides, or the replacement with a marker gene.
2) The modification of an expression control region (or expression control sequence) may be the mutation on the expression control region (or expression control sequence) through deletion, insertion, non-conservative or conservative substitution, or a combination thereof, or the replacement with a sequence having more diminished activity. The expression control region includes a promoter, an operator sequence, a sequence for encoding a ribosomal binding site, and a sequence for controlling the termination of transcription and translation, but is not limited thereto.
3) and 4) The modification of the amino acid sequence or the polynucleotide sequence may be the mutation on the sequence through deletion, insertion, nonconservative or conservative substitution, or a combination thereof in the amino acid sequence of the polypeptide or the polynucleotide sequence encoding the polypeptide, or a replacement with an amino acid sequence or polynucleotide sequence modified to have more diminished activity or an amino acid sequence or polynucleotide sequence modified to have no activity, so as to diminish activity of the polypeptide, but is not limited thereto. For example, the expression of the gene may be suppressed or attenuated by introducing mutation into the polynucleotide sequence to form a termination codon, but is not limited thereto.
5) The modification of a nucleotide sequence encoding an initiation codon or 5'-UTR region of the gene transcript encoding the polypeptide may be, for example, the substitution with a nucleotide sequence encoding, rather than an endogenous initiation codon, another initiation codon having a lower expression rate of the polypeptide, but is not limited thereto.
6) The introduction of an antisense oligonucleotide (e.g., antisense RNA) complementarily binding to the gene transcript encoding the polypeptide may be referred to, for example, a literature [Weintraub, H. et al., Antisense-RNA as a molecular tool for genetic analysis, Reviews - Trends in Genetics, Vol. 1(1) 1986].
7) The addition of a sequence complementary to the Shine-Dalgarno sequence of the gene encoding the polypeptide to the upstream of the Shine-Dalgarno sequence so as to form a secondary structure that makes the attachment of ribosomes impossible may be making mRNA translation impossible or reducing the rate thereof.

Further, 8) the addition of a promoter, which is to be reverse-transcribed, to the 3' end of the open reading frame (ORF) of the gene sequence encoding the polypeptide (reverse transcription engineering, RTE) may be making an antisense nucleotide complementary to the gene transcript encoding the polypeptide to diminish the activity of the polypeptide.

Another aspect of the present disclosure provides a method of producing L-tryptophan, the method including the step of culturing, in a medium, the L-tryptophan-producing microorganism of the genus *Corynebacterium* into which the foreign glnA protein or the polynucleotide encoding the same is introduced.

The method of producing L-tryptophan of the present disclosure may include the step of culturing, in a medium, a microorganism in which the foreign glnA protein or the *glnA* gene encoding the same is enhanced; or a microorganism which is genetically modified to further enhance the foreign glnA protein or the *glnA* gene encoding the same, and the microorganism is as described above.

The foreign glnA protein may have the increased glutamine synthetase activity, as compared to that of the wild-type microorganism of the genus *Corynebacterium.* Alternatively, the foreign glnA protein may not be endogenously expressed in the wild-type microorganism of the genus *Corynebacterium.*

As used herein the term "culturing" refers to growing the microorganism of the present disclosure in appropriately adjusted environment conditions. The culture procedure of the present disclosure may be performed according to appropriate media or culture conditions known in the art. Such culturing process may be easily adjusted and used by a person skilled in the art according to the selected microorganism. Specifically, the culturing may be in a batch type, a continuous type, and/or a fed-batch type, but is not limited thereto.

As used herein, the "medium" refers to a mixture containing, as main ingredients, nutrient materials required for culturing the microorganism of the present disclosure, wherein the medium supplies nutrient materials including water essential for survival and growth, growth factors, etc. Specifically, as for the media and other culture conditions used for culturing the microorganism of the present disclosure, any medium that is commonly used for culturing microorganisms may be used without particular limitation. However, the microorganism of the present disclosure may be cultured under aerobic conditions in a common medium containing appropriate carbon sources, nitrogen sources, phosphorus sources, inorganic compounds, amino acids, and/or vitamins, while controlling the temperature, pH, etc.

Specifically, a culture medium for microorganisms of the genus *Corynebacterium* may be found in a literature ["Manual of Methods for General Bacteriology" by the American Society for Bacteriology (Washington D.C., USA, 1981)].

In the present disclosure, the carbon source may include carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, etc. may be used. These nitrogen sources may be used alone or in a combination of two or more thereof, but are not limited thereto.

The phosphate sources may include potassium phosphate monobasic, potassium phosphate dibasic, and sodium-containing salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be included. These constituent ingredients or precursors may be added to the medium in a batch or continuous manner. However, the present disclosure is not limited thereto.

The pH of the medium may be adjusted by adding compounds, such as ammonium hydroxide, potassium hydroxide, ammonia, phosphoric acid, and sulfuric acid, to the medium in an appropriate manner during culturing of the microorganism of the present disclosure. In addition, an anti-foaming agent, such as a fatty acid polyglycol ester, may be added to suppress foam formation during culturing. In addition, oxygen or oxygen-containing gas may be injected into the medium to maintain the aerobic state of the medium, or no gas may be injected or nitrogen, hydrogen or carbon dioxide gas may be injected to maintain the anaerobic or nonaerobic state of the medium, but is not limited thereto.

In the culturing of the present disclosure, the culture temperature may be maintained at 20°C to 45°C, specifically 25°C to 40°C, and the culturing may be performed for about 10 hours to 160 hours, but is not limited thereto.

The L-tryptophan produced by culturing of the present disclosure may be released into the medium or may remain in cells.

The method of producing L-tryptophan of the present disclosure may further include the steps of preparing the microorganism of the present disclosure, preparing a medium for culturing the microorganism, or a combination of these steps (regardless of the order, in any order), for example, before the culturing step.

The method of producing L-tryptophan of the present disclosure may further include the step of recovering the L-tryptophan from the medium resulting from the culturing (medium in which culturing has been performed) or the cultured microorganism. The recovering step may be further included after the culturing step.

The recovering may be collecting a desired L-tryptophan by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, HPLC, and a combination of these methods may be used, and the desired L-tryptophan may be recovered from the medium or microorganism by using an appropriate method known in the art.

In addition, the method of producing L-tryptophan of the present disclosure may further include a purification step. The purification may be performed by using an appropriate method known in the art. In an exemplary embodiment, when the method of producing L-tryptophan of the present disclosure includes both the recovering step and the purification step, the recovering step and the purification step may be performed continuously or discontinuously regardless of the order, or may be performed simultaneously or integrated into one step, but is not limited thereto.

In the method of the present disclosure, the foreign glnA protein, polynucleotide, vector, microorganism, etc. are as described in other aspects.

Still another aspect of the present disclosure provides a composition for producing L-tryptophan, the composition including the microorganism of the genus *Corynebacterium,* into which the foreign glnA protein or the polynucleotide encoding the same is introduced; the medium in which the microorganism is cultured; or a combination thereof.

The composition of the present disclosure may further include any appropriate excipient that is commonly used in the composition for producing L-tryptophan, and examples of the excipient may include a preserving agent, a wetting agent, a dispersing agent, a suspending agent, a buffer, a stabilizing agent, an isotonic agent, etc., but are not limited thereto.

Still another aspect of the present disclosure provides a method of preparing the L-tryptophan-producing microorganism of the genus *Corynebacterium,* the method including the step of introducing the foreign glnA protein or the polynucleotide encoding the same.

The foreign glnA protein, the polynucleotide encoding the same, and the microorganism are as described above.

The preparation method may include the step of modifying the microorganism such that the microorganism has the increased glutamine synthetase activity, as compared to the endogenous activity. This includes introducing the foreign glnA protein into the microorganism of the genus *Corynebacterium.*

Still another aspect of the present disclosure provides use of the microorganism of the genus *Corynebacterium,* into which a foreign glnA protein or a polynucleotide encoding the same is introduced, in the production of L-tryptophan.

The foreign glnA protein, and the microorganism of the genus *Corynebacterium* are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1: Exploration and Selection of Gene Encoding Glutamine Synthetase (glnA)

An amino acid sequence of glnA protein derived from *Corynebacterium glutamicum* was used as a query sequence, and PSI-BLAST was performed based on NCBI and Kegg databases to identify candidate proteins expected to have the activity of biosynthesizing glutamine from glutamic acid and ammonia, genes encoding the same, and microorganisms possessing the same. Among them, 11 kinds of candidate proteins were selected, considering the biosafety level applicable to amino acid production and the availability of the produced amino acids, which are shown in Table 1 below.

**[Table 1]**

| No. | Name of strain | Protein Registration Number | Genome Registration Number | Biosafety level |
|---|---|---|---|---|
| 1 | *Aureibacillus halotolerans* | WP_133580410.1 | NZ_SNYJ01000007.1 | 1 |
| 2 | *Caryophanon tenue* | WP_066543527.1 | NZ_MASJ01000003.1 | 1 |
| 3 | *Peribacillus simplex* | WP_063236399.1 | NZ_CP017704.1 | 1 |
| 4 | *Carnobacterium divergens* | WP_119907903.1 | NZ_CP016843.1 | 1 |
| 5 | *Bacillus gobiensis* | WP_053602893.1 | NZ_CP012600.1 | 1 |
| 6 | *Carnobacterium inhibens* | WP_023178082.1 | NC_022606.1 | 1 |
| 7 | *Carnobacterium iners* | WP_085560080.1 | NZ_FOAH01000008.1 | 1 |
| 8 | *Lactiplantibacillus paraplantarum* | WP_003640333.1 | NZ_CP028423.1 | 1 |
| 9 | *Sulfolobus acidocaldarius* | WP_011278306.1 | NC_007181.1 | 1 |
| 10 | *Petroclostridium xylanilyticum* | WP_094551848.1 | NZ_NPML01000029.1 | 1 |
| 11 | *Bacillus subtilis* | WP_003231737.1 | NZ_JNCM01000035.1 | 1 |

### Example 2: Construction of plasmid for gene insertion

To insert genes into the chromosome of *Corynebacterium,* a plasmid was constructed using pDCM2 (US 2023-0134555 A1).

Chromosomal DNA of the wild-type *Corynebacterium glutamicum* ATCC13869 was used as a template, and the upstream region and the downstream region, where homologous recombination on the chromosome occurs, were amplified using a pair of primers of SEQ ID NO: 25 and SEQ ID NO: 26 and a pair of primers of SEQ ID NO: 27 and SEQ ID NO: 28, respectively, thereby obtaining respective gene fragments.

Solg^{™} Pfu-X DNA polymerase was used as polymerase, and PCR amplification was performed under conditions of denaturation at 95°C for 4 minutes, 27 cycles of denaturation at 95°C for 30 seconds, annealing at 60°C for 30 seconds, and polymerization at 72°C for 50 seconds, followed by polymerization at 72°C for 5 minutes.

The primer sequences used here are shown in Table 2 below.

**[Table 2]**

| SEQ ID NO. | Name of sequence | Sequence (5'-> 3') |
|---|---|---|
| 25 | HR1 F | aacgacggccagtgaattcGCTCGAATGCCTGACTGACA |
| 26 | HR1 R | gtttAGTACTaaaccggaagggccAAAGGACGACTTCACGGTTA |
| 27 | HR2 F | ccggtttAGTACTaaacaggaagagccATTGAGGATGCGAAACTGT |
| 28 | HR2 R | tgcatgcctgcaggtcgacGTAATCGAATCACCGGCCAG |

The upstream and downstream fragments of the region where homologous recombination on the chromosome occurs, obtained through the above process, and pDCM2 vector for chromosomal transformation which was digested with EcoRI and Sall restriction enzymes were cloned using a Gibson assembly method (DG Gibson et al., NATURE METHODS, VOL.6 NO.5, MAY 2009, NEBuilder HiFi DNA Assembly Master Mix) to obtain a recombinant plasmid. Cloning was performed by mixing a Gibson assembly reagent with respective gene fragments in the calculated molar quantity and then storing the mixture at 50°C for 1 hour. The recombinant plasmid constructed as above was named pDCM2-ΔTn.

### Example 3: Preparation of Microorganism of Genus Corynebacterium Introduced with Foreign glnA gene

### 3-1: Preparation of Microorganism Introduced with Aureibacillus halotolerans-derived glnA gene

To insert *A. halotolerans-derived glnA* gene selected in Example 1 into genomic DNA of *Corynebacterium glutamicum,* PCR was performed using the *glnA* gene of *A. halotolerans* strain, which was synthesized using the gene synthesis service of Bionics Co., Ltd., as a template, and a pair of primers of SEQ ID NO: 29 and SEQ ID NO: 30 to amplify the *A. halotolerans-derived glnA* gene (NZ_SNYJ01000007.1, SEQ ID NO: 2). Solg^{™} Pfu-X DNA polymerase was used as polymerase, and PCR amplification was performed under conditions of denaturation at 95°C for 2 minutes, 27 cycles of denaturation at 95°C for 20 seconds, annealing at 60°C for 40 seconds, and polymerization at 72°C for 1 minute, followed by polymerization at 72°C for 5 minutes.

Further, in order to obtain a PlysCm1 promoter (US 2023-0134555 A1) for enhancing the activity of glnA protein, PCR was performed using pDCM2-Tn::PlysCm1_pheA (US 2023-0134555 A1) as a template, and a pair of primers of SEQ ID NO: 31 and SEQ ID NO: 32. Solg^{™} Pfu-X DNA polymerase was used as polymerase, and PCR amplification was performed under conditions of denaturation at 95°C for 2 minutes, 27 cycles of denaturation at 95°C for 20 seconds, annealing at 60°C for 40 seconds, and polymerization at 72°C for 30 seconds, followed by polymerization at 72°C for 5 minutes.

The amplified PlysCm1 promoter region, the *A. halotolerans-derived glnA* gene fragment, and the pDCM2-ΔTn of Example 2 which was digested with Scal restriction enzyme were cloned using a Gibson assembly method to obtain a recombinant plasmid. Cloning was performed by mixing a Gibson assembly reagent with respective gene fragments in the calculated molar quantity and then storing the mixture at 50°C for 1 hour. The recombinant plasmid including the *A. halotolerans-*derived *glnA* gene constructed as above was named pDCM2-△Tn:: PlysCm1-glnA(A.ht).

The constructed pDCM2-ΔTn::PlysCm1-glnA(A.ht) plasmid was transformed into an L-tryptophan producing strain CM05-9157 (US 2023-0134555 A1) by electroporation (Appl. Microbiol.Biotechnol. (1999) 52 :541-545), and then through a secondary homologous recombination, a strain was obtained, in which one copy of PlysCm1-glnA(A.ht) was introduced between transposon genes on the chromosome. The corresponding genetic manipulation was confirmed by PCR using a pair of primers of SEQ ID NO: 33 and SEQ ID NO: 34 capable of amplifying the external region of the upstream and downstream regions for homologous recombination where the corresponding gene was inserted, and genome sequencing. The strain thus obtained, into which PlysCm1-glnA(A.ht) was introduced, was named CM05-9883.

### 3-2: Preparation of Microorganism Introduced with Caryophanon tenue-derived glnA gene

To insert *C. tenue*-derived *glnA* gene selected in Example 1 into genomic DNA of *Corynebacterium glutamicum,* PCR was performed using the *glnA* gene of *C*. *tenue* strain, which was synthesized using the gene synthesis service of Bionics Co., Ltd., as a template, and a pair of primers of SEQ ID NO: 35 and SEQ ID NO: 36 in the same manner as in Example 3-1 to amplify the *C*. tenue-derived *glnA* gene (NZ_MASJ01000003.1, SEQ ID NO: 4).

Further, in order to obtain a PlysCm1 promoter for enhancing the activity of glnA protein, PCR was performed using pDCM2-Tn::PlysCm1_pheA as a template, and a pair of primers of SEQ ID NO: 31 and SEQ ID NO: 37 in the same manner as in Example 3-1.

The amplified PlysCm1 promoter region, the *C*. *tenue*-derived *glnA* gene fragment, and the pDCM2-ΔTn of Example 2 which was digested with Scal restriction enzyme were cloned using a Gibson assembly method to obtain a recombinant plasmid. Cloning was performed by mixing a Gibson assembly reagent with respective gene fragments in the calculated molar quantity and then storing the mixture at 50°C for 1 hour. The recombinant plasmid including the *C. tenue-*derived *glnA* gene constructed as above was named pDCM2-ΔTn::PlysCm1-glnA(C.te).

The constructed pDCM2-ΔTn::PlysCm1-glnA(C.te) plasmid was transformed into a tryptophan producing strain CM05-9157 by electroporation, and then through a secondary homologous recombination, a strain was obtained, in which one copy of PlysCm1-glnA(C.te) was introduced between transposon genes on the chromosome. The corresponding genetic manipulation was confirmed by PCR using a pair of primers of SEQ ID NO: 33 and SEQ ID NO: 34 capable of amplifying the external region of the upstream and downstream regions for homologous recombination where the corresponding gene was inserted, and genome sequencing. The strain thus obtained, into which PlysCm1-glnA(C.te) was introduced, was named CM05-9885.

### 3-3: Preparation of Microorganism Introduced with Peribacillus simplex-derived glnA gene

To insert *P. simplex*-derived *glnA* gene selected in Example 1 into genomic DNA of *Corynebacterium glutamicum,* PCR was performed using the *glnA* gene of *P*. *simplex* strain, which was synthesized using the gene synthesis service of Bionics Co., Ltd., as a template, and a pair of primers of SEQ ID NO: 38 and SEQ ID NO: 39 in the same manner as in Example 3-1 to amplify the *P. simplex*-derived *glnA* gene (NZ_CP017704.1, SEQ ID NO: 6).

Further, in order to obtain a PlysCm1 promoter for enhancing the activity of glnA protein, PCR was performed using pDCM2-Tn::PlysCm1_pheA as a template, and a pair of primers of SEQ ID NO: 31 and SEQ ID NO: 40 in the same manner as in Example 3-1.

The amplified PlysCm1 promoter region, the *P. simple*x-derived *glnA* gene fragment, and the pDCM2-ΔTn of Example 2 which was digested with Scal restriction enzyme were cloned using a Gibson assembly method to obtain a recombinant plasmid. Cloning was performed by mixing a Gibson assembly reagent with respective gene fragments in the calculated molar quantity and then storing the mixture at 50°C for 1 hour. The recombinant plasmid including the *P. simplex*-derived *glnA* gene constructed as above was named pDCM2-ΔTn::PlysCm1-glnA(P.si).

The constructed pDCM2-ΔTn::PlysCm1-glnA(P.si) plasmid was transformed into a tryptophan producing strain CM05-9157 by electroporation, and then through a secondary homologous recombination, a strain was obtained, in which one copy of PlysCm1-glnA(P.si) was introduced between transposon genes on the chromosome. The corresponding genetic manipulation was confirmed by PCR using a pair of primers of SEQ ID NO: 33 and SEQ ID NO: 34 capable of amplifying the external region of the upstream and downstream regions for homologous recombination where the corresponding gene was inserted, and genome sequencing. The strain thus obtained, into which PlysCm1-glnA(P.si) was introduced, was named CM05-9879.

### 3-4: Preparation of Microorganism Introduced with Carnobacterium divergens-derived glnA gene

To insert C. *divergens-derived glnA* gene selected in Example 1 into genomic DNA of *Corynebacterium glutamicum,* PCR was performed using the *glnA* gene of C. *divergens* strain, which was synthesized using the gene synthesis service of Bionics Co., Ltd., as a template, and a pair of primers of SEQ ID NO: 41 and SEQ ID NO: 42 in the same manner as in Example 3-1 to amplify the *C*. *divergens-derived glnA* gene (NZ_CP016843.1, SEQ ID NO: 8).

Further, in order to obtain a PlysCm1 promoter for enhancing the activity of glnA protein, PCR was performed using pDCM2-Tn::PlysCm1_pheA as a template, and a pair of primers of SEQ ID NO: 31 and SEQ ID NO: 43 in the same manner as in Example 3-1.

The amplified PlysCm1 promoter region, the *C*. *divergens-derived glnA* gene fragment, and the pDCM2-ΔTn of Example 2 which was digested with Scal restriction enzyme were cloned using a Gibson assembly method to obtain a recombinant plasmid. Cloning was performed by mixing a Gibson assembly reagent with respective gene fragments in the calculated molar quantity and then storing the mixture at 50°C for 1 hour. The recombinant plasmid including the *C. divergens-*derived *glnA* gene constructed as above was named pDCM2-△Tn::PlysCm1-glnA(C.di).

The constructed pDCM2-ΔTn::PlysCm1-glnA(C.di) plasmid was transformed into a tryptophan producing strain CM05-9157 by electroporation, and then through a secondary homologous recombination, a strain was obtained, in which one copy of PlysCm1-glnA(C.di) was introduced between transposon genes on the chromosome. The corresponding genetic manipulation was confirmed by PCR using a pair of primers of SEQ ID NO: 33 and SEQ ID NO: 34 capable of amplifying the external region of the upstream and downstream regions for homologous recombination where the corresponding gene was inserted, and genome sequencing. The strain thus obtained, into which PlysCm1-glnA(C.di) was introduced, was named CM05-9877.

### 3-5: Preparation of Microorganism Introduced with Bacillus gobiensis-derived glnA gene

To insert *B*. *gobiensis-derived glnA* gene selected in Example 1 into genomic DNA of *Corynebacterium glutamicum,* PCR was performed using the *glnA* gene of *B*. *gobiensis* strain, which was synthesized using the gene synthesis service of Bionics Co., Ltd., as a template, and a pair of primers of SEQ ID NO: 44 and SEQ ID NO: 45 in the same manner as in Example 3-1 to amplify the *B*. *gobiensis-derived glnA* gene (NZ_CP012600.1, SEQ ID NO: 10).

Further, in order to obtain a PlysCm1 promoter for enhancing the activity of glnA protein, PCR was performed using pDCM2-Tn::PlysCm1_pheA as a template, and a pair of primers of SEQ ID NO: 31 and SEQ ID NO: 46 in the same manner as in Example 3-1.

The amplified PlysCm1 promoter region, the *B*. *gobiensis-derived glnA* gene fragment, and the pDCM2-ΔTn of Example 2 which was digested with Scal restriction enzyme were cloned using a Gibson assembly method to obtain a recombinant plasmid. Cloning was performed by mixing a Gibson assembly reagent with respective gene fragments in the calculated molar quantity and then storing the mixture at 50°C for 1 hour. The recombinant plasmid including the *B*. *gobiensis-*derived *glnA* gene constructed as above was named pDCM2-△Tn::PlysCm1-glnA(B.go).

The constructed pDCM2-ΔTn::PlysCm1-glnA(B.go) plasmid was transformed into a tryptophan producing strain CM05-9157 by electroporation, and then through a secondary homologous recombination, a strain was obtained, in which one copy of PlysCm1-glnA(B.go) was introduced between transposon genes on the chromosome. The corresponding genetic manipulation was confirmed by PCR using a pair of primers of SEQ ID NO: 33 and SEQ ID NO: 34 capable of amplifying the external region of the upstream and downstream regions for homologous recombination where the corresponding gene was inserted, and genome sequencing. The strain thus obtained, into which PlysCm1-glnA(B.go) was introduced, was named CM05-9879.

### 3-6: Preparation of Microorganism Introduced with Carnobacterium inhibens-derived glnA gene

To insert *C*. *inhibens-derived glnA* gene selected in Example 1 into genomic DNA of *Corynebacterium glutamicum,* PCR was performed using the *glnA* gene of *C*. *inhibens* strain, which was synthesized using the gene synthesis service of Bionics Co., Ltd., as a template, and a pair of primers of SEQ ID NO: 47 and SEQ ID NO: 48 in the same manner as in Example 3-1 to amplify the *C*. *inhibens-derived glnA* gene (NC_022606.1, SEQ ID NO: 12).

Further, in order to obtain a PlysCm1 promoter for enhancing the activity of glnA protein, PCR was performed using pDCM2-Tn::PlysCm1_pheA as a template, and a pair of primers of SEQ ID NO: 31 and SEQ ID NO: 49 in the same manner as in Example 3-1.

The amplified PlysCm1 promoter region, the *C*. *inhibens-derived glnA* gene fragment, and the pDCM2-ΔTn of Example 2 which was digested with Scal restriction enzyme were cloned using a Gibson assembly method to obtain a recombinant plasmid. Cloning was performed by mixing a Gibson assembly reagent with respective gene fragments in the calculated molar quantity and then storing the mixture at 50°C for 1 hour. The recombinant plasmid including the *C. inhibens-*derived *glnA* gene constructed as above was named pDCM2-△Tn::PlysCm1-glnA(C.in).

The constructed pDCM2-ΔTn::PlysCm1-glnA(C.in) plasmid was transformed into a tryptophan producing strain CM05-9157 by electroporation, and then through a secondary homologous recombination, a strain was obtained, in which one copy of PlysCm1-glnA(C.in) was introduced between transposon genes on the chromosome. The corresponding genetic manipulation was confirmed by PCR using a pair of primers of SEQ ID NO: 33 and SEQ ID NO: 34 capable of amplifying the external region of the upstream and downstream regions for homologous recombination where the corresponding gene was inserted, and genome sequencing. The strain thus obtained, into which PlysCm1-glnA(C.in) was introduced, was named CM05-9878.

### 3-7: Preparation of Microorganism Introduced with Carnobacterium iners-derived glnA gene

To insert *C. iners*-derived *glnA* gene selected in Example 1 into genomic DNA of *Corynebacterium glutamicum,* PCR was performed using the *glnA* gene of *C*. *iners* strain, which was synthesized using the gene synthesis service of Bionics Co., Ltd., as a template, and a pair of primers of SEQ ID NO: 50 and SEQ ID NO: 51 in the same manner as in Example 3-1 to amplify the *C. iners*-derived *glnA* gene (NZ_FOAH01000008.1, SEQ ID NO: 14).

Further, in order to obtain a PlysCm1 promoter for enhancing the activity of glnA protein, PCR was performed using pDCM2-Tn::PlysCm1_pheA as a template, and a pair of primers of SEQ ID NO: 31 and SEQ ID NO: 52 in the same manner as in Example 3-1.

The amplified PlysCm1 promoter region, the *C. iners*-derived *glnA* gene fragment, and the pDCM2-ΔTn of Example 2 which was digested with Scal restriction enzyme were cloned using a Gibson assembly method to obtain a recombinant plasmid. Cloning was performed by mixing a Gibson assembly reagent with respective gene fragments in the calculated molar quantity and then storing the mixture at 50°C for 1 hour. The recombinant plasmid including the *C. iners*-derived *glnA* gene constructed as above was named pDCM2-ΔTn::PlysCm1-glnA(C.ar).

The constructed pDCM2-ΔTn::PlysCm1-glnA(C.ar) plasmid was transformed into a tryptophan producing strain CM05-9157 by electroporation, and then through a secondary homologous recombination, a strain was obtained, in which one copy of PlysCm1-glnA(C.ar) was introduced between transposon genes on the chromosome. The corresponding genetic manipulation was confirmed by PCR using a pair of primers of SEQ ID NO: 33 and SEQ ID NO: 34 capable of amplifying the external region of the upstream and downstream regions for homologous recombination where the corresponding gene was inserted, and genome sequencing. The strain thus obtained, into which PlysCm1-glnA(C.ar) was introduced, was named CM05-9884.

### 3-8: Preparation of Microorganism Introduced with Lactiplantibacillus paraplantarum-derived glnA gene

To insert *L. paraplantarum-derived glnA* gene selected in Example 1 into genomic DNA of *Corynebacterium glutamicum,* PCR was performed using the *glnA* gene of *L. paraplantarum* strain, which was synthesized using the gene synthesis service of Bionics Co., Ltd., as a template, and a pair of primers of SEQ ID NO: 53 and SEQ ID NO: 54 in the same manner as in Example 3-1 to amplify the L. *paraplantarum-derived glnA* gene (NZ_CP028423.1, SEQ ID NO: 16).

Further, in order to obtain a PlysCm1 promoter for enhancing the activity of glnA protein, PCR was performed using pDCM2-Tn::PlysCm1_pheA as a template, and a pair of primers of SEQ ID NO: 31 and SEQ ID NO: 55 in the same manner as in Example 3-1.

The amplified PlysCm1 promoter region, the *L. paraplantarum-derived glnA* gene fragment, and the pDCM2-ΔTn of Example 2 which was digested with Scal restriction enzyme were cloned using a Gibson assembly method to obtain a recombinant plasmid. Cloning was performed by mixing a Gibson assembly reagent with respective gene fragments in the calculated molar quantity and then storing the mixture at 50°C for 1 hour. The recombinant plasmid including the *L. paraplantarum-*derived *glnA* gene constructed as above was named pDCM2-△Tn::PlysCm1-glnA(L.pa).

The constructed pDCM2-ΔTn::PlysCm1-glnA(L.pa) plasmid was transformed into a tryptophan producing strain CM05-9157 by electroporation, and then through a secondary homologous recombination, a strain was obtained, in which one copy of PlysCm1-glnA(L.pa) was introduced between transposon genes on the chromosome. The corresponding genetic manipulation was confirmed by PCR using a pair of primers of SEQ ID NO: 33 and SEQ ID NO: 34 capable of amplifying the external region of the upstream and downstream regions for homologous recombination where the corresponding gene was inserted, and genome sequencing. The strain thus obtained, into which PlysCm1-glnA(L.pa) was introduced, was named CM05-9880.

### 3-9: Preparation of Microorganism Introduced with Sulfolobus acidocaldarius-derived glnA gene

To insert *S*. *acidocaldarius-derived glnA* gene selected in Example 1 into genomic DNA of *Corynebacterium glutamicum,* PCR was performed using the *glnA* gene of S. *acidocaldarius* strain, which was synthesized using the gene synthesis service of Bionics Co., Ltd., as a template, and a pair of primers of SEQ ID NO: 56 and SEQ ID NO: 57 in the same manner as in Example 3-1 to amplify the S. *acidocaldarius-derived glnA* gene (NC_007181.1, SEQ ID NO: 18).

Further, in order to obtain a PlysCm1 promoter for enhancing the activity of glnA protein, PCR was performed using pDCM2-Tn::PlysCm1_pheA as a template, and a pair of primers of SEQ ID NO: 31 and SEQ ID NO: 58 in the same manner as in Example 3-1.

The amplified PlysCm1 promoter region, the *S*. *acidocaldarius-derived glnA* gene fragment, and the pDCM2-ΔTn of Example 2 which was digested with Scal restriction enzyme were cloned using a Gibson assembly method to obtain a recombinant plasmid. Cloning was performed by mixing a Gibson assembly reagent with respective gene fragments in the calculated molar quantity and then storing the mixture at 50°C for 1 hour. The recombinant plasmid including the *S*. *acidocaldarius-*derived *glnA* gene constructed as above was named pDCM2-△Tn::PlysCm1-glnA(S.ac).

The constructed pDCM2-ΔTn::PlysCm1-glnA(S.ac) plasmid was transformed into a tryptophan producing strain CM05-9157 by electroporation, and then through a secondary homologous recombination, a strain was obtained, in which one copy of PlysCm1-glnA(S.ac) was introduced between transposon genes on the chromosome. The corresponding genetic manipulation was confirmed by PCR using a pair of primers of SEQ ID NO: 33 and SEQ ID NO: 34 capable of amplifying the external region of the upstream and downstream regions for homologous recombination where the corresponding gene was inserted, and genome sequencing. The strain thus obtained, into which PlysCm1-glnA(S.ac) was introduced, was named CM05-9882.

### 3-10: Preparation of Microorganism Introduced with Petroclostridium xylanilyticum-derived glnA gene

To insert *P. xylanilyticum-derived glnA* gene selected in Example 1 into genomic DNA of *Corynebacterium glutamicum,* PCR was performed using the *glnA* gene of *P. xylanilyticum* strain, which was synthesized using the gene synthesis service of Bionics Co., Ltd., as a template, and a pair of primers of SEQ ID NO: 59 and SEQ ID NO: 60 in the same manner as in Example 3-1 to amplify the P. *xylanilyticum-derived glnA* gene (NZ_NPML01000029.1, SEQ ID NO: 20).

Further, in order to obtain a PlysCm1 promoter for enhancing the activity of glnA protein, PCR was performed using pDCM2-Tn::PlysCm1_pheA as a template, and a pair of primers of SEQ ID NO: 31 and SEQ ID NO: 61 in the same manner as in Example 3-1.

The amplified PlysCm1 promoter region, the *P. xylanilyticum-derived glnA* gene fragment, and the pDCM2-ΔTn of Example 2 which was digested with Scal restriction enzyme were cloned using a Gibson assembly method to obtain a recombinant plasmid. Cloning was performed by mixing a Gibson assembly reagent with respective gene fragments in the calculated molar quantity and then storing the mixture at 50°C for 1 hour. The recombinant plasmid including the *P. xylanilyticum-*derived *glnA* gene constructed as above was named pDCM2-△Tn::PlysCm1-glnA(P.xy).

The constructed pDCM2-ΔTn::PlysCm1-glnA(P.xy) plasmid was transformed into a tryptophan producing strain CM05-9157 by electroporation, and then through a secondary homologous recombination, a strain was obtained, in which one copy of PlysCm1-glnA(P.xy) was introduced between transposon genes on the chromosome. The corresponding genetic manipulation was confirmed by PCR using a pair of primers of SEQ ID NO: 33 and SEQ ID NO: 34 capable of amplifying the external region of the upstream and downstream regions for homologous recombination where the corresponding gene was inserted, and genome sequencing. The strain thus obtained, into which PlysCm1-glnA(P.xy) was introduced, was named CM05-9886.

### 3-11: Preparation of Microorganism Introduced with Bacillus subtilis-derived glnA gene

To insert *B*. *subtilis-derived glnA* gene selected in Example 1 into genomic DNA of *Corynebacterium glutamicum,* PCR was performed using the *glnA* gene of *B*. *subtilis* strain, which was synthesized using the gene synthesis service of Bionics Co., Ltd., as a template, and a pair of primers of SEQ ID NO: 62 and SEQ ID NO: 63 in the same manner as in Example 3-1 to amplify the *B*. *subtilis-derived glnA* gene (NZ_JNCM01000035.1, SEQ ID NO: 22).

Further, in order to obtain a PlysCm1 promoter for enhancing the activity of glnA protein, PCR was performed using pDCM2-Tn::PlysCm1_pheA as a template, and a pair of primers of SEQ ID NO: 31 and SEQ ID NO: 64 in the same manner as in Example 3-1.

The amplified PlysCm1 promoter region, the *B*. *subtilis-derived glnA* gene fragment, and the pDCM2-ΔTn of Example 2 which was digested with Scal restriction enzyme were cloned using a Gibson assembly method to obtain a recombinant plasmid. Cloning was performed by mixing a Gibson assembly reagent with respective gene fragments in the calculated molar quantity and then storing the mixture at 50°C for 1 hour. The recombinant plasmid including the *B*. *subtilis-derived glnA* gene constructed as above was named pDCM2-ΔTn::PlysCm1-glnA(B.su).

The constructed pDCM2-ΔTn::PlysCm1-glnA(B.su) plasmid was transformed into a tryptophan producing strain CM05-9157 by electroporation, and then through a secondary homologous recombination, a strain was obtained, in which one copy of PlysCm1-glnA(B.su) was introduced between transposon genes on the chromosome. The corresponding genetic manipulation was confirmed by PCR using a pair of primers of SEQ ID NO: 33 and SEQ ID NO: 34 capable of amplifying the external region of the upstream and downstream regions for homologous recombination where the corresponding gene was inserted, and genome sequencing. The strain thus obtained, into which PlysCm1-glnA(B.su) was introduced, was named CM05-9550.

The primer sequences used in Example 3-1 to Example 3-11 are shown in Table 3 below.

**[Table 3]**

| SEQ ID NO. | Name of sequence | Sequence (5'-> 3') |
|---|---|---|
| 29 | glnA(A.ht)-F | gaaaggtgcacacatATGGGTTCTAAGTACACGAG |
| 30 | glnA(A.ht)-R | CTCTTCCTGTTTAGTTTAGTAAAGGGTAAGGTACTGC |
| 31 | PlysCm1-F | CCCTTCCGGTTTAGTGCTCCTTAGGGAGCCATCTTTTG |
| 32 | PlysCm1(A.ht)-R | GTACTTAGAACCCATatgtgtgcacctttcgatct |
| 33 | Confirm-PlysCm1-glnA-F | TTTACCTCCCACAAGTCC |
| 34 | Confirm-PlysCm1-glnA-R | AAGAATCTCCCTGCCAAA |
| 35 | glnA(C.te)-F | aaggtgcacacatATGGGTAAATACACAAAAGACG |
| 36 | glnA(C.te)-R | CTCTTCCTGTTTAGTTTAATACATTTTTAAATATTGGTCACG TTCC |
| 37 | PlysCm1(C.te)-R | TGTGTATTTACCCATatgtgtgcacctttcgatct |
| 38 | glnA(P.si)-F | AAAGGTGCACAcatATGGCAAAGTTCACACGTGAA |
| 39 | glnA(P.si)-R | CTCTTCCTGTTTAGTTTAATACATTGACATATATTGTTCGCG |
| 40 | PlysCm1(P.si)-R | TGTGAACTTTGCCATatgtgtgcacctttcgatct |
| 41 | glnA(C.di)-F | aaggtgcacacatATGCCAGAATTTACAAGAGAAC |
| 42 | glnA(C.di)-R | CTCTTCCTGTTTAGTTTAGTATAACTCTAAATATTGTTCTCT |
| 43 | PlysCm1(C.di)-R | TGTAAATTCTGGCATatgtgtgcacctttcgatct |
| 44 | glnA(B.go)-F | ggtgcacacatATGGCAAAGTATACTAGAGAAGAT |
| 45 | glnA(B.go)-R | CTCTTCCTGTTTAGTTTAAAACTGAGTTAAATATTGATCGCG T |
| 46 | PlysCm1(B.go)-R | AGTATACTTTGCCATatgtgtgcacctttcgatct |
| 47 | glnA (C.in)-F | ATGAAAAAATTTACAAGGAAAGAAATCGAAATAAGTGC |
| 48 | glnA (C.in)-R | CTCTTCCTGTTTAGTTTAataTAAGGTTAGATATTTTTCTTTTT CCCATTCAGAAACTTG |
| 49 | PlysCm1((C.in)-R | TGTAAATTTTTTCATatgtgtgcacctttcgatct |
| 50 | glnA(C.ar)-F | ATGAAAAATTTTACTAGAGAAGAAATCGTAACAAG |
| 51 | glnA(C.ar)-R | CTCTTCCTGTTTAGTTTAATAAAGAGTCATATACTTTTCTTG TTCCC |
| 52 | PlysCm1(C.ar)-R | AGTAAAATTTTTCATatgtgtgcacctttcgatct |
| 53 | glnA(L.pa)-F | gaaaggtgcacacatATGGCAAAACAGTCCTATTC |
| 54 | glnA(L.pa)-R | CTCTTCCTGTTTAGTTTAATACAGTTCCATGTACTGATCACG T |
| 55 | PlysCm1(L.pa)-R | GGACTGTTTTGCCATatgtgtgcacctttcgatct |
| 56 | glnA(S.ac)-F | aaggtgcacacatATGCCAGGACTTCCAAAAAATG |
| 57 | glnA(S.ac)-R | CTCTTCCTGTTTAGTTTAAGAATCTAGATACTGATATATTTC CATAGGATGTGG |
| 58 | PlysCm1(S.ac)-R | TGGAAGTCCTGGCATatgtgtgcacctttcgatct |
| 59 | glnA(P.xy)-F | gaaaggtgcacacatATGCCTAACTATACCAGGGA |
| 60 | glnA(P.xy)-R | CTCTTCCTGTTTAGTTTAATATCTGGTGAGATACTGGTCG |
| 61 | PlysCm1(P.xy)-R | GGTATAGTTAGGCATatgtgtgcacctttcgatct |
| 62 | glnA(B.su)-F | GAAAGGTGCACACATATGGCAAAGTACACTAGAGAAG |
| 63 | glnA(B.su)-R | CTCTTCCTGTTTAGTTTAATACTGAGACATATACTGTTCG |
| 64 | PlysCm1(B.su)-R | AGTGTACTTTGCCATATGTGTGCACCTTTCGATCTA |

### Comparative Example: Preparation of Microorganism of the genus Corynebacterium Introduced with Corynebacterium glutamicum-derived glnA gene

As a comparative example, the *glnA* gene derived from the wild-type *Corynebacterium glutamicum* ATCC 13869 was inserted into genomic DNA of *Corynebacterium glutamicum,* and the nucleotide sequence of the start codon was replaced with ATG to increase the expression level of glnA protein. First, PCR was performed using the genomic DNA of the wild-type *Corynebacterium glutamicum* ATCC 13869 strain as a template and a pair of primers of SEQ ID NO: 65 and SEQ ID NO: 66 in the same manner as in 3-1 to amplify the *glnA* gene (SEQ ID NO: 23) derived from the wild-type *Corynebacterium glutamicum* ATCC 13869.

Further, in order to obtain a PlysCm1 promoter for enhancing the activity of glnA protein, PCR was performed using pDCM2-Tn::PlysCm1_pheA as a template, and a pair of primers of SEQ ID NO: 31 and SEQ ID NO: 67 in the same manner as in Example 3-1.

The amplified PlysCm1 promoter region, the wild-type *Corynebacterium glutamicum* ATCC 13869-derived *glnA* gene fragment, and the pDCM2-βTn of Example 2 which was digested with Scal restriction enzyme were cloned using a Gibson assembly method to obtain a recombinant plasmid. Cloning was performed by mixing a Gibson assembly reagent with respective gene fragments in the calculated molar quantity and then storing the mixture at 50°C for 1 hour. The recombinant plasmid including the wild-type *Corynebacterium glutamicum* ATCC 13869-derived *glnA* gene constructed as above was named pDCM2-βTn::PlysCm1-glnA(C.gl).

The constructed pDCM2-βTn::PlysCm1-glnA(C.gl) plasmid was transformed into a tryptophan producing strain CM05-9157 by electroporation, and then through a secondary homologous recombination, a strain was obtained, in which one copy of PlysCm1-glnA(C.gl) was introduced between transposon genes on the chromosome. The corresponding genetic manipulation was confirmed by PCR using a pair of primers of SEQ ID NO: 33 and SEQ ID NO: 34 capable of amplifying the external region of the upstream and downstream regions for homologous recombination where the corresponding gene was inserted, and genome sequencing. The strain thus obtained, into which PlysCm1-glnA(C.gl) was introduced, was named CM05-9548.

The primer sequences used in Comparative Example are shown in Table 4 below.

**[Table 4]**

| SEQ ID NO. | Name of sequence | Sequence (5'-> 3') |
|---|---|---|
| 65 | glnA(C.gl)-F | GAAAGGTGCACACATATGGCGTTTGAAACCCCGGA |
| 66 | glnA(C.gl)-R | CTCTTCCTGTTTAGTTTAGCAGTCGAAGTACAATTCGAA |
| 67 | PlysCm1(C.gl)-R | GGTTTCAAACGCCATATGTGTGCACCTTTCGATCTACG |

### Example 4: Verification of L-Tryptophan-Producing ability of Microorganisms of the genus Corynebacterium Introduced with foreign glnA gene

To examine L-tryptophan producing abilities of the strains prepared in Examples 3-1 to 3-11, in which each foreign glnA gene was introduced, the parent strain CM05-9157 as a control, and CM05-9548 strain into which the *glnA* gene derived from the wild-type *Corynebacterium glutamicum* ATCC 13869 was introduced, they were cultured as follows.

Each strain was inoculated into a 250 mL corner-baffle flask containing 25 mL of a seed medium, and cultured with shaking at 200 rpm for 20 hours at 30°C to obtain each seed culture. 1 mL of the seed culture was inoculated into a 250 mL corner-baffle flask containing 25 mL of a production medium, and cultured with shaking at 200 rpm for 24 hours at 30°C. After the completion of culturing, the production amount of L-tryptophan was measured using HPLC, and the results are shown in Table 5 below.

### <Seed Medium>

20 g of glucose, 10 g of peptone, 5 g of yeast extract, 1.5 g of urea, 4 g of KH₂PO₄, 8 g of K₂HPO₄, 0.5 g of MgSO₄ 7H₂O, 100 µg of biotin, 1000 µg of thiamine HCl, 2000 µg of calcium pantothenate, 2000 µg of nicotinamide, pH 7.0 (based on 1 L of distilled water)

### <Production Medium>

30 g of glucose, 15 g of (NH₄)₂SO₄, 1.2 g of MgSO₄ 7H₂O, 1 g of KH₂PO₄, 5 g of yeast extract, 900 µg of biotin, 4500 µg of thiamine hydrochloride, 4500 µg of calcium pantothenate, 30 g of CaCO₃, pH 7.0 (based on 1 L of distilled water).

**[Table 5]**

| Name of strain | Strain of origin of introduced *glnA* gene | OD562 | Production amount of L-tryptophan (g/L) | Yield of L-tryptophan (*100 g/g, %) |
|---|---|---|---|---|
| CM05-9157 | - | 56.9 | 1.89 | 6.37 |
| CM05-9548 | *Corynebacterium glutamicum* | 57 | 1.88 | 6.36 |
| CM05-9877 | *Carnobacterium divergens* | 56.5 | 1.91 | 6.45 |
| CM05-9878 | *Carnobacterium inhibens* | 56.5 | 1.9 | 6.4 |
| CM05-9879 | *Peribacillus simplex* | 52.5 | 2.17 | 7.3 |
| CM05-9880 | *Lactiplantibacillus paraplantarum* | 57.2 | 1.77 | 5.96 |
| CM05-9881 | *Bacillus gobiensis* | 57.4 | 1.74 | 5.86 |
| CM05-9882 | *Sulfolobus acidocaldarius* | 58 | 1.64 | 5.51 |
| CM05-9883 | *Aureibacillus halotolerans* | 51.9 | 2.37 | 7.93 |
| CM05-9884 | *Carnobacterium iners* | 56.8 | 1.88 | 6.35 |
| CM05-9885 | *Caryophanon tenue* | 52 | 2.33 | 7.77 |
| CM05-9886 | *Petroclostridium* | 57.1 | 1.76 | 5.9 |
| | *xylanilyticum* | | | |
| CM05-9550 | *Bacillus subtilis* | 53.1 | 1.97 | 6.68 |

As shown in Table 5, the tryptophan production of CM05-9548 strain, into which the *glnA* gene derived from *Corynebacterium glutamicum* was introduced, was 1.88 g/L, which was almost equal to that of the parent strain CM05-9157 strain, into which the *glnA* gene was not introduced.

In contrast, among 11 kinds of production strains, into which the foreign glnA gene was introduced, CM05-9883 strain into which the *A. halotolerans-derived glnA* gene was introduced produced a final 2.37 g/L of L-tryptophan in the flask culture, showing about 25% improvement in the fermentation yield, as compared to the control CM05-9548 strain. CM05-9885 strain into which the *C*. tenue-derived *glnA* gene was introduced produced final 2.33 g/L of L-tryptophan in the flask culture, showing about 23% improvement in the fermentation yield, as compared to the control CM05-9548 strain. Further, CM05-9879 strain into which the *P. simplex*-derived *glnA* gene was introduced produced final 2.17 g/L of L-tryptophan in the flask culture, showing about 15% improvement in the fermentation yield, as compared to the control CM05-9548 strain.

These results confirmed that among 11 kinds of foreign *glnA* genes, the *A. halotolerans-, C. tenue-,* and *P. simplex*-derived *glnA* genes specifically improved the L-tryptophan producing ability in the *Corynebacterium glutamicum* strain.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A microorganism of the genus *Corynebacterium,* into which a foreign glnA protein or a polynucleotide encoding the same is introduced.

2. The microorganism of claim 1, wherein the protein is derived from any one or more microorganisms selected from the group consisting of *Aureibacillus halotolerans, Caryophanon tenue,* and *Peribacillus simplex.*

3. The microorganism of claim 1, wherein the protein includes any one amino acid sequence of SEQ ID NOS: 1, 3, and 5, or any one or more selected from the group consisting of amino acid sequences having 70% or more sequence identity thereto.

4. The microorganism of claim 1, wherein the microorganism of the genus *Corynebacterium* has an increased L-tryptophan producing ability, as compared to the microorganism of the genus *Corynebacterium* before modification.

5. The microorganism of claim 1, wherein the microorganism of the genus *Corynebacterium* is *Corynebacterium glutamicum.*

6. A method of producing L-tryptophan, the method comprising the step of culturing, in a medium, a microorganism of the genus *Corynebacterium,* into which a foreign glnA protein or a polynucleotide encoding the same is introduced.

7. The method of claim 6, wherein the protein is derived from any one or more microorganisms selected from the group consisting of *Aureibacillus halotolerans, Caryophanon tenue,* and *Peribacillus simplex.*

8. A composition for producing L-tryptophan, the composition comprising a microorganism of the genus *Corynebacterium,* into which a foreign glnA protein or a polynucleotide encoding the same is introduced; a medium in which the microorganism is cultured; or a combination thereof.

9. The composition of claim 8, wherein the protein is derived from any one or more microorganisms selected from the group consisting of *Aureibacillus halotolerans, Caryophanon tenue,* and *Peribacillus simplex.*
